# EUROPEAN PATENT SPECIFICATION

(11) **EP 0 938 295 B1**
(45) Date of publication and mention of the grant of the patent: **29.09.2004**
(21) Application number: 97930367.4
(22) Date of filing: 16.07.1997
(51) Int. Cl.: A61K 9/107, A61K 33/06, A61K 9/00

(54) **A NON-AQUEOUS ORALLY ADMINISTRABLE, PASTE-LIKE COMPOSITION FOR VETERINARY USE, A METHOD FOR THE PREPARATION THEREOF, AND THE USE OF AN OILY VEHICLE FOR THE MANUFACTURE OF SUCH COMPOSITION**
NICHT-WÄSSRIGES ORAL VERABREICHBARES PASTENFÖRMIGES MITTEL ZUR TIERÄRZTLICHEN VERWENDUNG, VERFAHREN ZU DESSEN HERSTELLUNG SOWIE DIE VERWENDUNG EINES ÖLIGEN TRÄGERS ZUR HERSTELLUNG EINES SOLCHEN MITTELS
COMPOSITION NON-AQUEUSE PATEUSE ADMINISTRABLE PER OS A USAGE VETERINAIRE, SON PROCEDE DE PREPARATION ET EMPLOI D'UN SUPPORT HUILEUX POUR SA FABRICATION

(30) Priority: 16.07.1996 DK 79196
(43) Date of publication of application: 01.09.1999
(73) Proprietor: JORGEN KRUUSE A/S, 5290 Marslev (DK)
(72) Inventor: AGGER, Nicolai, DK-5800 Nyborg (DK); ZANGENBERG, Niels, Hoenberg, DK-1365 Copenhagen K (DK); CHRISTENSEN, Finn, Norring, DK-2970 Hoersholm (DK)
(74) Representative: Kjerrumgaard, Bent
(86) International application number: PCT/DK1997/000313
(87) International publication number: WO 1998/002143

(56) References cited:
- EP-A- 0 667 152
- WO-A-90/09797
- STN INTERNATIONAL, File CAPLUS, CAPLUS Accession No. 1996:255504, GOFF J.P. et al., "Field Trials of an Oral Calcium Propionate Paste as an Aid to Prevent Milk Fever in Periparturient Dairy Cows"; & J. DAIRY SCI., (1996), 79(3), 378-83.
- DIALOG INFORMATION SERVICES, File 5, BIOSIS, Dialog Accession No. 9586346, Biosis Accession No. 94091346, WENTINK G.H. et al., "Oral Administration of Calcium Chloride-Containing Products Testing for Deleterious Side-Effects"; & VET. Q., 14(2), 1992, 76-79.

## Description

The present invention relates to an orally administrable, paste-like composition for veterinary use, in particular for ruminants, and more particularly a composition comprising a calcium compound for controlling milk fever (parturient paresis).

The invention also relates to the use of a particular oily vehicle for the manufacture of such a composition, as well as a method for the preparation of such a composition.

In spite of several decades of research, milk fever is still common among highly productive cows, and it is a substantial economical problem for milk producers. Moreover, sub-clinical milk fever occurs five to ten times as often as clinical milk fever, cf Goff, JP et al; The Pathophysiology and Prevention of Milk Fever; Vet Med 1987; September; 943-950. The economical problem is intensified by the fact that the risk of supervening diseases attacking the cow is substantially increased at the time when she is ready for producing milk, cf Curtis, CR et al; Association of Parturient Hypocalcemia with Periparturient Disorders in Holstein Cows, JAVMA 1983; 183; 559-574. Conditions such as mastitis, ketosis, dystokia, retained afterbirth, inversion of uterus, and metritis occur more frequently in cows having hypocalcemia in connection with the calving than in normal cows.

Oral administration of calcium at the strategically correct times in connection with the calving to prevent milk fever has been practiced for many years. The preferred calcium source is calcium chloride because it is readily available, has a high content of calcium, and calcium is easily absorbed from calcium chloride. However, aqueous solutions of calcium chloride have a bitter taste and a locally irritating effect on the walls of the cow's rumen. In an attempt to avoid these drawbacks, other calcium sources have been investigated.

For example a commercially available product contains calcium propionate as a gel on a vegetable oil basis. While calcium propionate does not have the same bitter taste and irritating effect as calcium chloride, it is relatively poorly absorbed into circulation compared to calcium chloride. Gels of calcium chloride may mask the bitter taste, but can have an etching effect on the cow's rumen (cf Wentink et van den Ingh; Vet Quarterly; Vol 14; no. 2; April 1992).

EP-B1-0 460 080 describes the use of a water-in-oil emulsion comprising a water soluble, readily absorbable calcium salt, preferably calcium chloride, dissolved in the aqueous phase, which is emulsified in an edible oil. Because the calcium chloride is dissolved in the aqueous phase of the water-in-oil emulsion, the etching effect of calcium chloride is substantially reduced. However, cows do not willingly take a liquid preparation, and there is a risk that some of the product will be spilt.

Paste-like compositions for the treatment of various ruminant diseases, such as ketosis and acidosis, are known and can be administered by a particular administration system, Bovivet, which comprises a plastic tube which is placed with its tip at the posterior part of the cow's tongue, where the paste-like content is emptied concurrently with the cow's swallowing movements. A satisfactory paste-like composition of a calcium compound for the treatment of milk fever has not yet been available.

It is an object of the present invention to provide an orally administrable, paste-like composition for veterinary use comprising a suspension of a particulate, veterinarily active compound in an oily vehicle, which composition is useful for the administration of a veterinarily active compound, in particular a calcium compound for treatment of milk fever.

The composition of the invention is defined in claim 1.

It has surprisingly been found that a particulate, veterinarily active compound can form a stable suspension of a paste-like nature in such a two-phase, oily vehicle. Without wishing to be bound by any theory, it is assumed that particles of the compound to be suspended adhere to the surfaces of the fat crystals forming. a coherent structure in the liquid oil component.

The orally administrable, paste-like composition of the present invention may comprise a suspension of any particulate, veterinarily active compound which it may be desirable to administer as a paste, for example by means of the above-mentioned Bovivet system. Most preferred, the particulate, veterinarily active compound is one which it would be of interest to have "embedded", either because of an ill or bitter taste, and/or because of an irritating effect to the animal's stomach. An example of a compound which could be formulated into the orally administrable composition of the present invention would be an antibiotic which is bitter-tasting and affect the bacterial flora, for example in the cow's rumen. Another example of a veterinarily active compound which it could be of interest to administer by way of the present invention would be a pain-relieving compound, for example in connection with mastitis.

The present invention is of particular interest when the particulate, veterinarily active compound is a calcium compound such as calcium chloride, calcium propionate, calcium formiate or calcium oxide, in particular calcium chloride. When calcium chloride is formulated by means of the two-phase oil system, it has surprisingly been found that the bitter taste can be reduced and that there will be no etching effect on the rumen. Moreover, surprisingly, calcium is readily absorbed from a calcium chloride-containing composition of the invention so that it can effectively control milk fever. Although Applicant does not wish to be bound by any theory, it is believed that a non-aqueous composition of the invention containing particles of calcium chloride activate the oesophageal groove, which leads the composition to the abomasum without passing thorugh the rumen. This can explain why an orally administered calcium chloride-containing composition of the invention has no etching effect on the wall of the rumen and can easily be absorbed into the blood stream of the cow.

In the treatment of milk fever it is often desirable to administer a magnesium compound together with a calcium compound. When the particulate, veterinarily active compound in the composition of the invention is a calcium compound, the composition may therefore further comprise a magnesium compound, preferably magnesium chloride and/or magnesium oxide.

The paste-like nature of the composition of the invention is obtained by the selection of the oil and fat components, the amount of particulate, veterinarily active compound, and the particle size. If the amount of particulate compound is too high, the composition becomes too viscous and looses its paste-like nature. In order that the composition retains its paste-like nature, the upper limit of the particulate, veterinarily active compound should preferably not exceed 75 %, preferably 70% (w/w). If the amount of particulate compound is too low, the composition takes the nature of a thin suspension and becomes too liquid for retaining its paste-like properties. Preferably the amount of particulate, veterinarily active compound is not below 50%, preferably not below 60%, and most preferably not below 65 % (w/w). The composition should preferably retain its paste-like consistency during storage up to a temperature of 45 °C, and preferably down to 0° C, more preferably from 3° to 30°C.

As mentioned above, the oily vehicle to be used according to the invention is a two-phase oil consisting of a liquid oil component and a solid fat component which forms a coherent structure of crystals in the liquid component. Two-phase oils of this type are known and have previously been used as liquid frying oils or oleomargarines and as meat spreads for barbecuing. A two-phase oily system of the nature here contemplated has not previously been suggested as a vehicle for a veterinarily active composition.

Therefore, in another aspect, the invention also relates to the use as a vehicle for a veterinarily active composition of a two-phase oily composition comprising a liquid, veterinarily acceptable oil component and a solid, veterinarily acceptable fat component, said fat component forming a coherent structure of crystals in said liquid oil component.

The liquid oil component is preferably a vegetable oil or a fish oil. The liquid oil component should be selected so that it is liquid at its use temperature, preferably from 0° to 25 °C. The liquid oil should also be selected among such oils which do not dissolve the solid fat crystals. Based on such considerations, the preferred liquid oils are rapeseed oil, cotton seed oil, corn oil, mustard seed oil, sunflower oil, safflower oil, sesame oil, soybean oil, peanut oil and olive oil. Rapeseed oil is presently the most preferred liquid oil component.

The solid fat component is one which forms a coherent structure of crystals in the liquid oil component. Preferably the solid fat component is of vegetable origin manufactured from hardened vegetable oils, such as mixtures of palm oil and rapeseed oil, or mixtures of soybean oil and sunflower oil. Preferably the solid fat component comprises partially saturated triglycerides, diglycerides and/or monoglycerides, most preferred triglycerides, which are crystalline below the use temperature of the composition. Preferably the fat glycerides are selected to have melting points above 50°C. They therefore belong to the group of high-melting fats.

The relative amounts of liquid oil component and solid fat component in the two-phase oily vehicle should be such that the oily vehicle has a thick viscosity similar to vaseline at room temperature and still is pourable. The preferred consistency is obtained when the oily vehicle comprises from 6 to 20% (w/w) of the solid fat component.

The two-phase oily vehicle is manufactured by melting the liquid oil components and the solid fat components together at a temperature of for example 100°C. Hereby the components should form a fully miscible liquid solution. This solution is cooled very quickly, preferably within a few seconds from a temperature around 60-70°C to 20°C. Such a rapid cooling may be performed by means of a scrape surface heat exchanger, wherefrom the oily vehicle may be tapped as a thick, pumpable liquid at 20°C. The solid fat component crystallizes on standing and may be dissolved again upon heating. The oily vehicle should preferably be stored at a temperature below room temperature.

The orally administrable, paste-like composition of the invention may be prepared by a method comprising the step of mixing an oily vehicle comprising a first phase consisting of a liquid, veterinarily acceptable oil component and a second phase consisting of a solid veterinarily acceptable fat component, said fat component forming a coherent structure of crystals in said liquid oil component and a particulate, veterinarily active compound. In practice, the oily vehicle is stirred in a mixer at room temperature, whereby it becomes thinner like a thixotropic liquid. The particulate, veterinarily active compound may be added at any time, and the whole mixture should be stirred until the particles of the active compound have been evenly distributed in the oily vehicle. The mixture may then be drained off the mixer and allowed to settle. It is believed that the fat crystals form an interactive structure of crystals, whereto the salt adheres, and wherein the liquid oil component fills the voids. When examined by confical microscopy, the structure will appear like a sponge, wherein the oil fills the voids and the fat forms the solid structure.

The particulate, veterinally active compound may have any particle size which is useful having regard to factors such as absorption and viscosity of the composition. When the active compound is calcium chloride, any form of calcium chloride may be used such as anhydrous CaCl₂. CaCl₂,2H₂O and CaCl₂,6H₂O.

CaCl₂.H₂O is presently preferred in a particle size of about 360 to 370 µ.

In addition to a calcium compound and a magnesium compound, an orally administrable composition of the invention may further comprise liquid paraffin or paraffin oil, which can improve the consistency of the composition so that it is easier swallowed by a cow. Also paraffin seems to have its own masking effect upon the bitter taste of calcium chloride. To further improve the taste, the composition may comprise flavour additives such as lemon oil, sweeteners, such as artificial sweeteners, and antioxidants such as propylgallat.

An oral composition according to the invention is a socalled nutraceutical because it contains nutrients which, when given at the proper time in the proper amounts and with the proper intervals, will have a curing effect on the treated animal. The composition of the invention may further be a pharmaceutical when the active component is a pharmaceutically active compound such as an antibiotic or a pain-releasing compound. The composition of the invention may further be a nutrient, when the active particulate compound has a nutritive effect.

The invention will now be further described with reference to examples.

### EXAMPLE 1

A two-phase oily vehicle for use in accordance with the invention was supplied by Århus Oliefabrik A/S under the sample specification: OFD metal 2908-2. It contained 90% rapeseed oil and 10% hardened vegetable oil (the fat component). The amount of free fatty acid (determined as per cent oleic acid) was specified as maximally 0.1 % ab factory. The peroxide number (meq/kg) was specified as maximally 0.5 ab factory. The penetration (15°C) was specified as 270. To 100 g of this oil was added 183 g CaCl₂, 2H₂O (corresponding to 50 g Ca+ +), 5.8 g MgCl₂, 2H₂O, 0.3 g flavour additives and 200 ppm antioxidant.

The composition manufactured had a paste-like nature and could easily be administered to cows.

The above composition was modified by adding 6 g paraffin oil and reducing the amount of flavour additives from 0.3 to 0.2 g (by avoiding lemon oil). The composition obtained an improved consistency and was easily administered to cows.

### EXAMPLE 2

The first-mentioned composition of example 1 was subjected to a pharmacokinetic examination as follows.

Four Danish cattle-treating veterinarians were asked to find one or two herds of Danish black-and-white, dairy breed cattle where two cows calving for the third or fourth time in each herd were to calve at intervals of a few days. The cows were not to have shown any signs of milk fever at any of their previous calvings.

The cow who calved first in each herd was selected as test cow. Thus, the second cow in each herd was the control cow.

In order to establish basic values (before and after calving), heparin-stabilized blood samples of all cows to be tested were taken no later than 24 hours before expected calving and no sooner than 36 hours after calving.

The test cows received oral doses of calcium paste at the following times:
- First time:: 1 tube 10 to 14 hours before expected calving.
- Second time:: 1 tube in connection with calving.
- Third time:: 1 tube approximately 12 hours after calving.
- Fourth time:: 1 tube approximately 24 hours after calving.

Blood samples were taken at the following times:

Immediately before dosing, 30 minutes after, 1 hour after, and 2 to six hours after dosing.

The control cows received no treatment. Only in cases where clinical milk fever was observed, the control cows received intravenous treatment with calcium.

The blood samples for the control cow were as often as possible taken at the same times with relation to the calving time as the blood samples for the test cow of the herd. In case of clinical milk fever, additional blood samples were taken immediately before intravenous calcium treatment, 30 minutes after and 2 to 4 hours after treatment.

The blood samples were analysed for total calcium on VetTest® 8008 (Idexx Labs. Inc.), which is a machine for dry chemical blood analysis in veterinary practice. All samples were determined in duplicate.

### Results

Seven Danish black-and-white, dairy breed herds were selected. The oral dosing of the seven test cows was in general performed without difficulties and with no waste of calcium paste. Only one cow fought against the administration, and a few blobs of calcium paste were spilt.

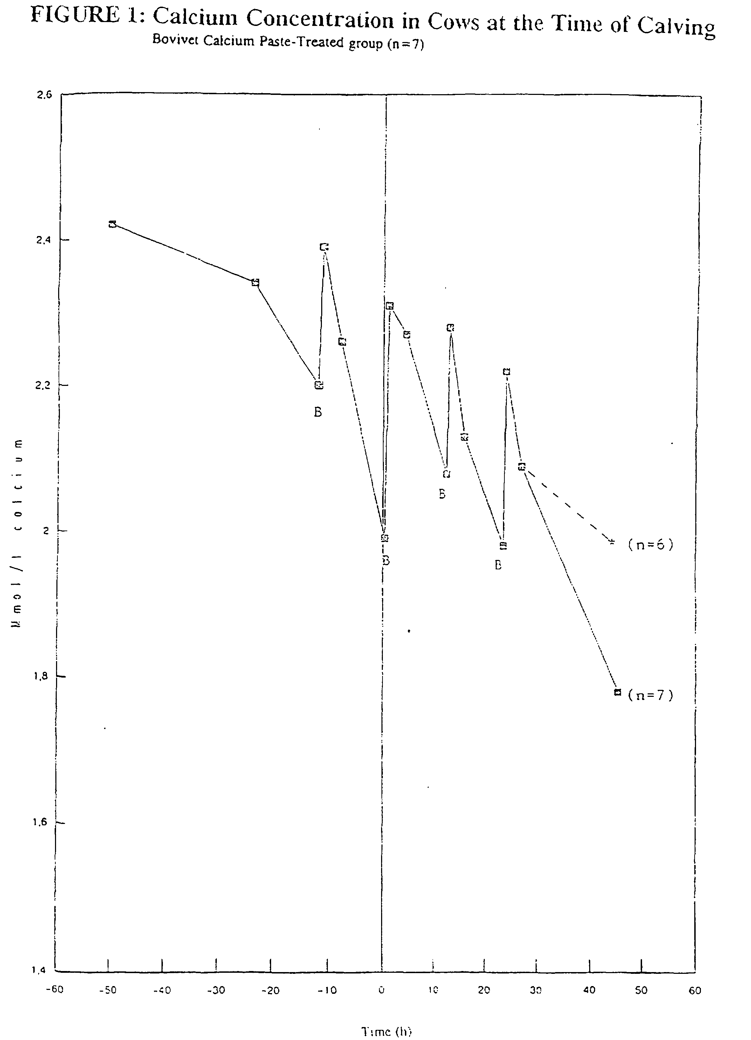

The average plasma concentration of calcium in the test cows is illustrated in figure 1. The letters **B** shown in the figure indicate the (average) times of the four oral calcium dosages. One test cow had milk fever approximately 48 hours after calving and was treated therapeutically with calcium intravenously.

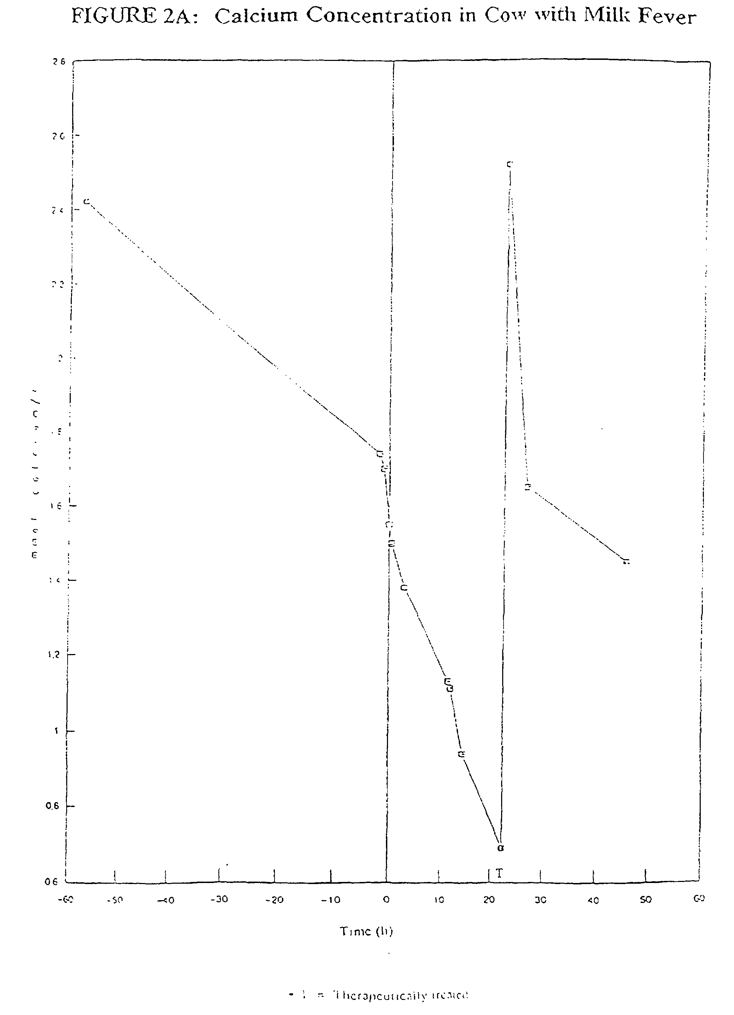

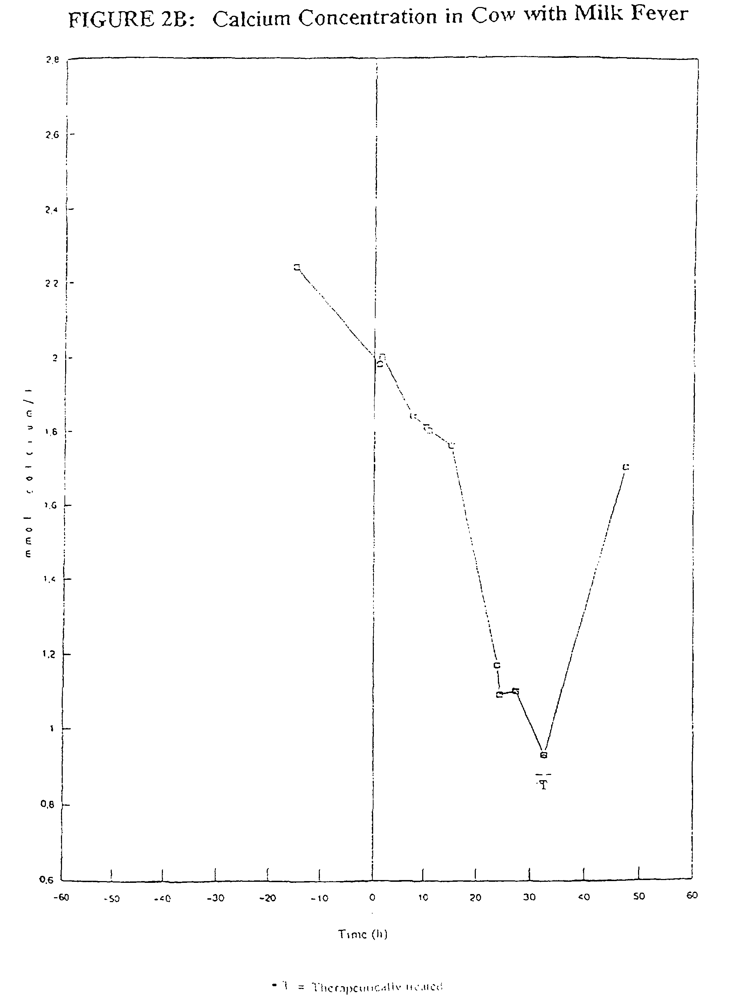

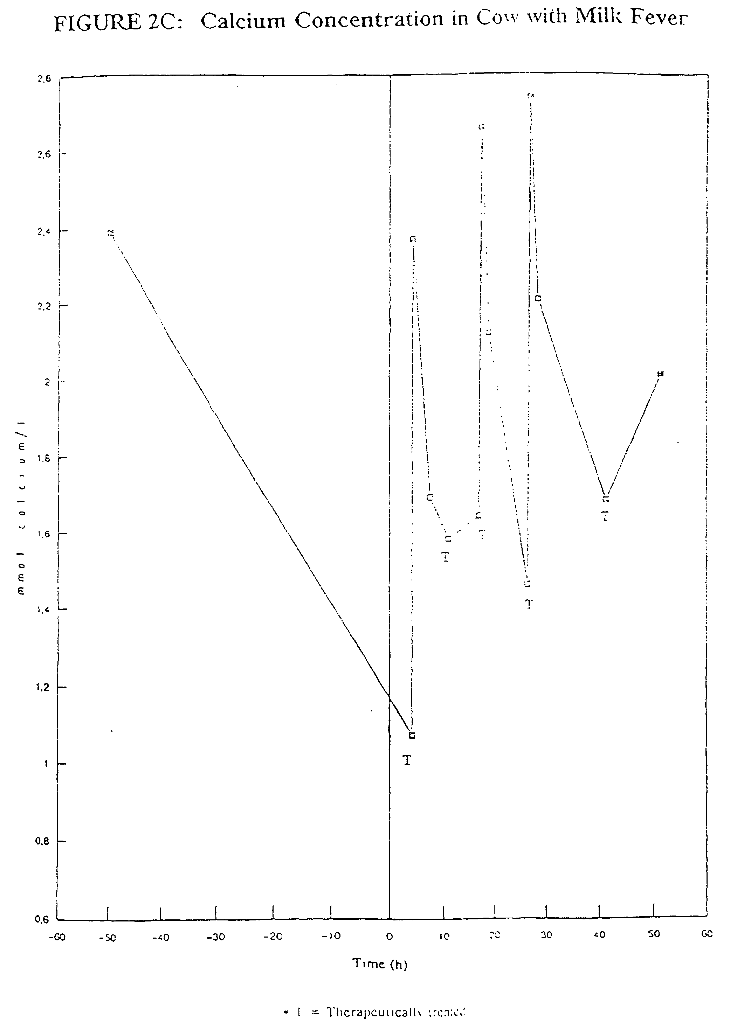

Three of seven control cows had milk fever and were treated therapeutically with calcium intravenously. One of the cows had several relapses and had to be treated curatively with calcium intravenously five times in all before she recovered. The actual calcium concentrations for the three cows suffering from milk fever are shown in figures 2A, 2B and 2C.

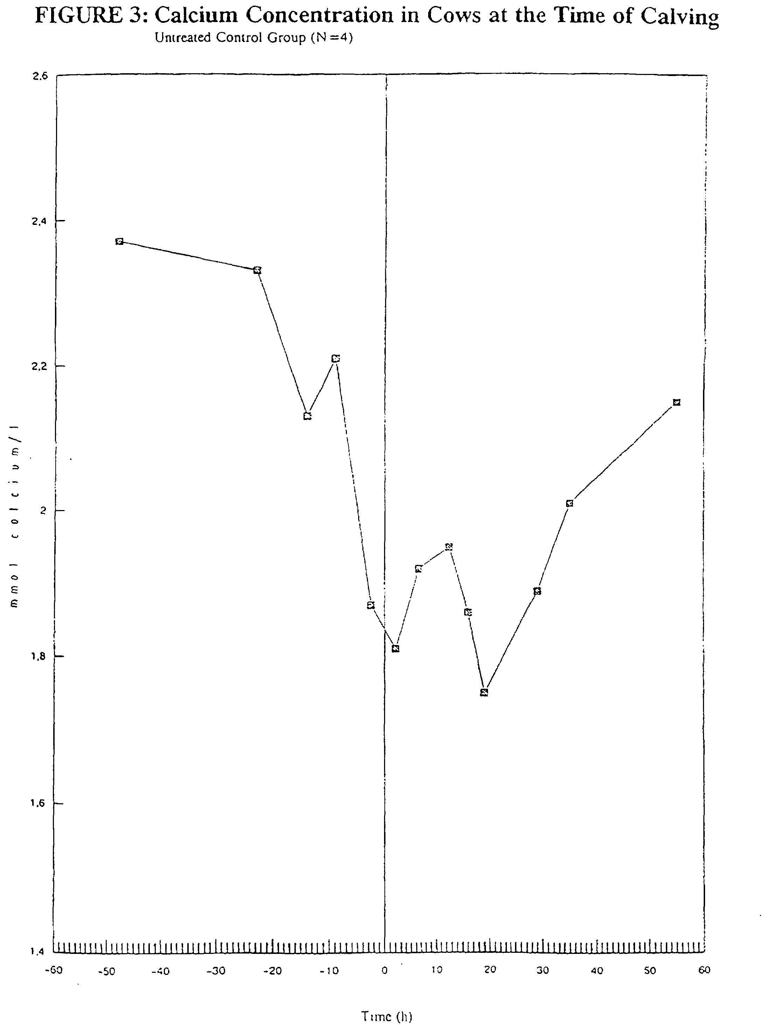

The calving of the remaining four control cows took place without any difficulties, and the average calcium concentration for these cows is shown in figure 3.

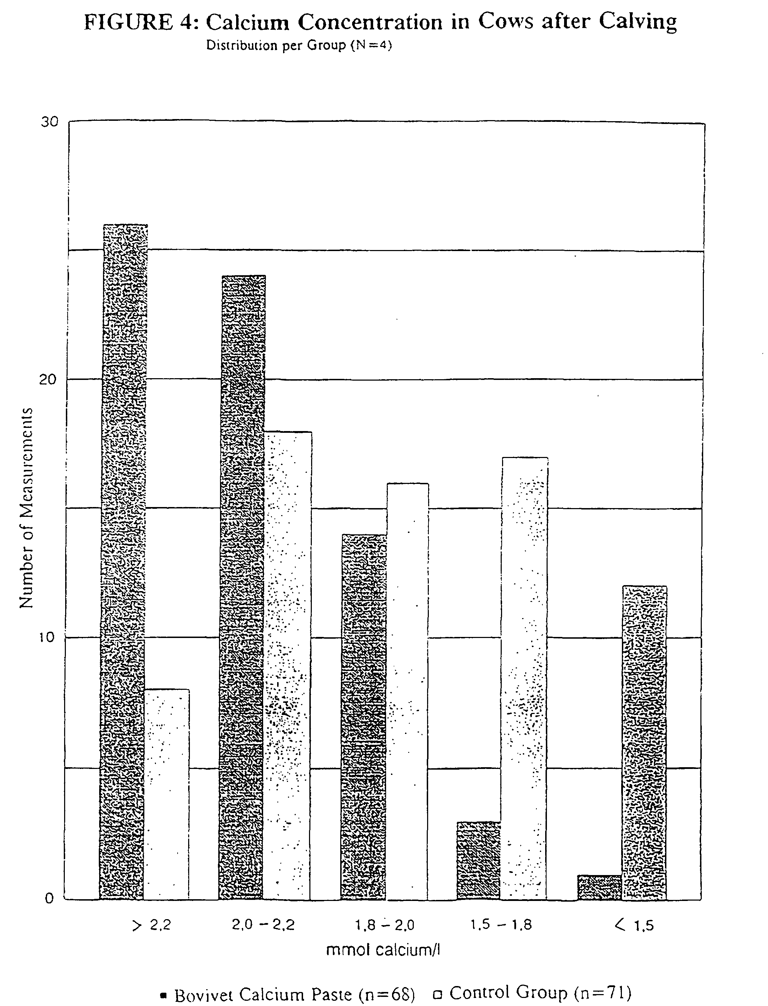

The individual calcium measurements per group after calving have in figure 4 been made up for the intervals:
> 2.2 2.0-2.2 1.8-2.0 1.5-1.8 < 1.5 mmol Ca/l

### Discussion and Conclusion

This test shows that the paste-like formulation according to the invention, in which the calcium chloride is embedded in a specially prepared two-phase oil, provides increased calcium concentrations at the time of calving when the formulation is administered four times at intervals of approximately 12 hours, beginning approximately 12 hours before expected calving.

Moreover, the test has shown that it has been difficult for the participating treatments were given at widely dispersed times. Four of the seven test cows were treated at an average of 17 hours before calving (11 to 29 hours before), whereas the remaining three test cows were treated at an average 4 hours before calving (2.5 to 5 hours before).

One test cow had milk fever 48 hours after calving, which is 24 hours after the last calcium treatment. The calcium value for this cow immediately before therapeutical treatment was 0.53 mmol Ca/l. If this measurement is not included in the average number by the last measurement for the test group (cf figure 1), the calcium concentration would have been 1.99 mmol Ca/l.

From figure 4 it appears that by means of the preventive program, 73.5 % of the registered calcium measurements after calving are higher than 2.0 mmol calcium/l. 5.9% of the measurements are below 1.8 mmol calcium/l. The respective numbers for the control group are 36.6% and 40.8%, respectively.

However, the tendency can be observed that the calcium concentration in the treated cows does not return to the normal concentration as rapidly as is the case for the untreated cows. This may be due to the fact that the cow's own calcium mobilization is hindered by the intensive calcium treatment. Similar cases have been observed in human patients with potassium deficiencies. An intensive potassium treatment hinders the patient's own potassium mobilization. If this intensive treatment is phased down at the end of the treatment period, the patient's own potassium mobilization will take over, which eliminates a transitional period of hypocalcemia.

By a comparison of the above-mentioned observations it is possible to provide the following proposal for a new preventive program against milk fever by means of the newly developed calcium paste with 183 g calcium chloride and 5.8 g magnesium chloride per tube:
- First dosage:: 1 tube 4 to 8 hours before expected calving
- Second dosage:: 1 tube around the time of calving
- Third dosage:: 1 tube 10 to 12 hours after calving
- Fourth dosage:: 1/2 tube 20 to 24 hours after calving
- Fifth dosage:: 1/2 tube 30 to 40 hours after calving

This program considers the interests of both the attending veterinarian and/or farmer and the calver. The attending veterinarian may easier determine the first treatment time, and the calver's own calcium mobilization will at first be dominated by an intensive calcium treatment and later on be supported by a less intensive calcium treatment.

The proposed program should be tested in a controlled clinical testing of one or more herds in which many cases of milk fever occur.

## Claims

1. A non-aqueous orally administrable, paste-like composition for veterinary use comprising a suspension of a particulate, veterinarily active calcium compound in an oily vehicle, said vehicle comprising a first phase consisting of a liquid, veterinarily acceptable oil component and a second phase consisting of a solid, veterinarily acceptable fat component, said fat component forming a coherent structure of crystals in said liquid oil component obtainable by stirring at room temperature said oily vehicle to obtain a thixotropic-like liquid.

2. Orally administrable composition according to claim 1, wherein the calcium compound is calcium chloride, calcium propionate, calcium formiate or calcium oxide.

3. Orally administrable composition according to claim 2, wherein the calcium compound is calcium chloride.

4. Orally administrable composition according to claims 1-3, further comprising a magnesium compound.

5. Orally administrable composition according to claim 4, wherein said magnesium compound is magnesium chloride and/or magnesium oxide.

6. Orally administrable composition according to claims 1-5, wherein the amount of particulate, veterinarily active compound is from 65% to 70% (w/w).

7. Orally administrable composition according to claims 1-6, wherein said liquid oil component is a vegetable oil or a fish oil.

8. Orally administrable composition according to claim 7, which is of paste-like consistency at a temperature of from 0 to 45° C, preferably from 3 to 30° C.

9. Orally administrable composition according to claim 7 or claim 8, wherein said liquid oil component is rapeseed oil, cotton seed oil, corn oil, mustard seed oil, sunflower oil, safflower oil, sesame oil, soybean oil, peanut oil or olive oil.

10. Orally administrable composition according to claims 1-9, wherein said solid fat component is of vegetable origin.

11. Orally administrable composition according to claim 10, wherein said solid fat component comprises partially saturated triglycerides, diglycerides and/or monoglycerides.

12. Orally administrable composition according to claim 11, wherein said glycerides have melting points above 50°C.

13. Orally administrable composition according to claims 1-12, wherein the oily vehicle comprises from 6% to 20% (w/w) of the solid fat component.

14. Orally administrable composition according to claims 1-13, which further comprises liquid parafin (parafin oil).

15. Orally administrable composition according to claims 1-14, further comprising one or more components selected from the group comprising antioxidants, flavour additives and sweeteners.

16. The use as a vehicle for a non-aqueous veterinarily active calcium composition of a two-phase oily composition comprising a liquid, veterinarily acceptable oil component and a solid, veterinarily acceptable fat component, said fat component forming a coherent structure of crystals in said liquid oil component.

17. Use according to claim 16, wherein the veterinarily active calcium composition is a nutraceutical, pharmaceutical or nutrient.

18. Use according to claim 17. wherein the veterinarily active calcium composition is for ruminants.

19. Use according to claim 18, wherein the calcium component is calcium chloride.

20. A method for the preparation of a non-aqueous orally administrable, paste-like composition for veterinary use, comprising the step of mixing an oily vehicle comprising a first phase consisting of a liquid, veterinarily acceptable oil component and a second phase consisting of a solid veterinarily acceptable fat component, said fat component forming a coherent structure of crystals in said liquid oil component and a particulate, veterinarily active calcium compound.

## Patentansprüche

1. Nichtwässerige, oral verabreichbare, pastenförmige Zusammensetzung zur veterinärmedizinischen Verwendung, umfassend eine Suspension aus einer teilchenförmigen, veterinärmedizinisch aktiven Calciumverbindung in einem öligen Träger, welcher Träger eine erste Phase bestehend aus einer flüssigen veterinärmedizinisch annehmbaren Ölkomponente und eine zweite Phase bestehend aus einer festen veterinärmedizinisch annehmbaren Fettkomponente aufweist, wobei die Fettkomponente eine kohärente Struktur von Kristallen in der flüssigen Ölkomponente bildet, die durch Rühren des öligen Trägers bei Raumtemperatur zur Erzielung einer Art thixotropen Flüssigkeit erhalten werden kann.

2. Oral verabreichbare Zusammensetzung nach Anspruch 1, worin die Kalziumverbindung Calciumchlorid, Calciumpropionat, Calciumformiat oder Calciumoxid ist.

3. Oral verabreichbare Zusammensetzung nach Anspruch 2, worin die Kalziumverbindung Calciumchlorid ist.

4. Oral verabreichbare Zusammensetzung nach den Ansprüchen 1 bis 3, welche weiters eine Magnesiumverbindung enthält.

5. Oral verabreichbare Zusammensetzung nach Anspruch 4, worin die Magnesiumverbindung Magnesiumchlorid und/oder Magnesiumoxid ist.

6. Oral verabreichbare Zusammensetzung nach den Ansprüchen 1 bis 5, worin die Menge an teilchenförmiger veterinärmedizinisch aktiver Verbindung 65 bis 70 % (w/w) ausmacht.

7. Oral verabreichbare Zusammensetzung nach den Ansprüchen 1 bis 6, worin die flüssige Ölkomponente ein pflanzliches Öl oder ein Fischöl ist.

8. Oral verabreichbare Zusammensetzung nach Anspruch 7, die bei einer Temperatur von 0 bis 45 °C, vorzugsweise 3 bis 30 °C, pastenförmige Konsistenz aufweist.

9. Oral verabreichbare Zusammensetzung nach Anspruch 7 oder 8, worin die flüssige Ölkomponente Rapsöl, Baumwollöl, Maisöl, Senföl, Sonnenblumenöl, Distelöl, Sesamöl, Sojaöl, Erdnussöl oder Olivenöl ist.

10. Oral verabreichbare Zusammensetzung nach den Ansprüchen 1 bis 9, worin die feste Fettkomponente pflanzlichen Ursprungs ist.

11. Oral verabreichbare Zusammensetzung nach Anspruch 10, worin die feste Fettkomponente teilweise gesättigte Triglyceride, Diglyceride und/oder Monoglyceride enthält.

12. Oral verabreichbare Zusammensetzung nach Anspruch 11, worin die Glyceride einen Schmelzpunkt von über 50 °C aufweisen.

13. Oral verabreichbare Zusammensetzung nach den Ansprüchen 1 bis 12, worin der ölige Träger 6 bis 20 % (w/w) der festen Fettkomponente aufweist.

14. Oral verabreichbare Zusammensetzung nach den Ansprüchen 1 bis 13, welche weiters flüssiges Paraffin (Paraffinöl) enthält.

15. Oral verabreichbare Zusammensetzung nach den Ansprüchen 1 bis 14, welche weiters eine oder mehr Komponenten ausgewählt aus der Gruppe umfassend Antioxidantien, Geschmackszusätze und Süßungsmittel enthält.

16. Verwendung eines Trägers für eine nichtwässerige veterinärmedizinisch aktive Calciumzusammensetzung einer öligen Zweiphasen-Zusammensetzung umfassend eine flüssige veterinärmedizinisch annehmbare Ölkomponente und eine feste veterinärmedizinisch annehmbare Fettkomponente, wobei die Fettkomponente eine kohärente Struktur von Kristallen in der flüssigen Ölkomponente bildet.

17. Verwendung nach Anspruch 16, worin die veterinärmedizinisch aktive Calciumzusammensetzung eine Nahrungsergänzung, ein Pharmazeutikum oder ein Nahrungsmittel ist.

18. Verwendung nach Anspruch 17, worin die veterinärmedizinisch aktive Calciumzusammensetzung für Wiederkäuer bestimmt ist.

19. Verwendung nach Anspruch 18, worin die Calciumkomponente Calciumchlorid ist.

20. Verfahren zur Herstellung einer nichtwässerigen, oral verabreichbaren, pastenförmigen Zusammensetzung zur vetrerinärmedizinischen Verwendung, welches den Schritt des Mischens eines öligen Trägers enthaltend eine erste Phase bestehend aus einer flüssigen veterinärmedizinisch annehmbaren Ölkomponente und eine zweite Phase bestehend aus einer festen veterinärmedizinisch annehmbaren Fettkomponente, wobei die Fettkomponente eine kohärente Struktur von Kristallen in der flüssigen Ölkomponente bildet, und einer teilchenförmigen veterinärmedizinisch aktiven Calciumverbindung umfasst.

## Revendications

1. Composition non aqueuse, de type pâte, administrable par voie orale, pour une utilisation vétérinaire comprenant une suspension d'un composé de calcium particulaire actif sur le plan vétérinaire dans un excipient huileux, ledit excipient comprenant une première phase comprenant un composant huileux liquide acceptable sur le plan vétérinaire et une deuxième phase comprenant un composant gras solide acceptable sur le plan vétérinaire, ledit composant gras formant une structure cohérente de cristaux dans ledit composant huileux liquide pouvant être obtenue en agitant à température ambiante ledit excipient huileux pour obtenir un liquide de type thixotrope.

2. Composition administrable par voie orale suivant la revendication 1, dans laquelle le composé de calcium est du chlorure de calcium, du propionate de calcium, du formiate de calcium ou de l'oxyde de calcium.

3. Composition administrable par voie orale suivant la revendication 2, dans laquelle le composé de calcium est du chlorure de calcium.

4. Composition administrable par voie orale suivant les revendications 1-3, comprenant en outre un composé de magnésium.

5. Composition administrable par voie orale suivant la revendication 4, dans laquelle ledit composé de magnésium est du chlorure de magnésium et/ou de l'oxyde de magnésium.

6. Composition administrable par voie orale suivant les revendications 1-5, dans laquelle la quantité de composé particulaire actif sur le plan vétérinaire est de 65% à 70% (p/p).

7. Composition administrable par voie orale suivant la revendication 1-6, dans laquelle ledit composant huileux liquide est une huile végétale ou une huile de poisson.

8. Composition administrable par voie orale suivant la revendication 7, qui est d'une consistance de type pâte à une température de 0 à 45°C, de préférence de 3 à 30°C.

9. Composition administrable par voie orale suivant la revendication 7 ou la revendication 8, dans laquelle ledit composant huileux liquide est de l'huile de colza, de l'huile de coton, de l'huile de maïs, de l'huile de moutarde, de l'huile de tournesol, de l'huile de carthame, de l'huile de sésame, de l'huile de soja, de l'huile d'arachide ou de l'huile d'olive.

10. Composition administrable par voie orale suivant les revendications 1-9, dans laquelle ledit composant gras solide est d'origine végétale.

11. Composition administrable par voie orale suivant la revendication 10, dans laquelle ledit composant gras solide comprend des triglycérides, des diglycérides et/ou des monoglycérides partiellement saturés.

12. Composition administrable par voie orale suivant la revendication 11, dans laquelle lesdits glycérides présentent des points de fusion supérieurs à 50°C.

13. Composition administrable par voie orale suivant les revendications 1-12, dans laquelle l'excipient huileux comprend de 6% à 20% (p/p) du composant gras solide.

14. Composition administrable par voie orale suivant les revendications 1-13, qui comprend en outre de la paraffine liquide (huile de paraffine).

15. Composition administrable par voie orale suivant les revendications 1-14, comprenant en outre un ou plusieurs composants sélectionnés parmi le groupe comprenant des antioxydants, des additifs d'arôme et des édulcorants.

16. Utilisation d'un excipient pour une composition de calcium non aqueuse active sur le plan vétérinaire d'une composition huileuse à deux phases comprenant un composant huileux liquide acceptable sur le plan vétérinaire et un composant gras solide acceptable sur le plan vétérinaire, ledit composant gras formant une structure cohérente de cristaux dans ledit composant huileux liquide.

17. Utilisation suivant la revendication 16, dans laquelle la composition de calcium active sur le plan vétérinaire est un alicament, un médicament ou un nutriment.

18. Utilisation suivant la revendication 17, dans laquelle la composition de calcium active sur le plan vétérinaire est destinée à des ruminants.

19. Utilisation suivant la revendication 18, dans laquelle le composant de calcium est du chlorure de calcium.

20. Procédé pour la préparation d'une composition non aqueuse de type pâte administrable par voie orale pour une utilisation vétérinaire, comprenant l'étape de mélanger un excipient huileux comprenant une première phase comprenant un composant huileux liquide acceptable sur le plan vétérinaire et une deuxième phase comprenant un composant gras solide acceptable sur le plan vétérinaire, ledit composant gras formant une structure cohérente de cristaux dans ledit composant huileux liquide et un composant de calcium particulaire actif sur le plan vétérinaire.
